(19) Europäisches Patentamt
European Patent Office
Office européen des brevets

(11) **EP 3 878 937 A1**

(12) **EUROPEAN PATENT APPLICATION**

(43) Date of publication:
**15.09.2021 Bulletin 2021/37**

(51) Int Cl.:
***C12M 1/32*** *(2006.01)*  ***C12M 1/00*** *(2006.01)*
***C12M 1/09*** *(2006.01)*  ***C12M 1/12*** *(2006.01)*
***C12M 1/34*** *(2006.01)*

(21) Application number: **21159321.5**

(22) Date of filing: **25.02.2021**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.03.2020 JP 2020043340**
**06.07.2020 JP 2020116651**
**22.01.2021 JP 2021008978**

(71) Applicant: **Ricoh Company, Ltd.**
**Tokyo 143-8555 (JP)**

(72) Inventors:
• **KOSHIZUKA, Shinnosuke**
**Tokyo, 143-8555 (JP)**

• **ARATANI, Tomoyuki**
**Tokyo, 143-8555 (JP)**
• **SAMESHIMA, Tatsuya**
**Tokyo, 143-8555 (JP)**
• **NAKAYAMA, Tomoaki**
**Tokyo, 143-8555 (JP)**
• **MIYAOKA, Atsushi**
**Tokyo, 143-8555 (JP)**
• **SHIONOIRI, Momoko**
**Tokyo, 143-8555 (JP)**
• **KITAZAWA, Tomofumi**
**Tokyo, 143-8555 (JP)**

(74) Representative: **SSM Sandmair**
**Patentanwälte Rechtsanwalt**
**Partnerschaft mbB**
**Joseph-Wild-Straße 20**
**81829 München (DE)**

(54) **DRYING SUPPRESSION DEVICE AND CELL CULTURE KIT**

(57) A drying suppression device including: a main body housing a well plate; and an openable/closable lid provided on the main body, the main body including: a holder section that holds the well plate; a liquid storage tank that stores liquid; and a support section that supports the holder while keeping the holder section away from an inner bottom surface of the liquid storage tank, wherein, with the lid being closed, an inner space surrounded by the liquid storage tank and the lid communicate with an outside space.

FIG. 2

**Description**

BACKGROUND OF THE INVENTION

Field of the Invention

The present invention relates to a drying suppression device and a cell culture kit.

Description of the Related Art

Currently, technological development is underway to replace a part of clinical tests conventionally performed by animal experiments with in vitro tests using live cells. In particular, the progress of stem cell technology has prompted studies on tests using human-derived stem cells as live cells.

In the in vitro tests using live cells, for example, live cells and a culture medium are placed in wells of a well plate, and a reagent to be evaluated is added before culturing the live cells. The effect of the reagent on the live cells is evaluated in terms of the shape of, connections between the cells, etc. of the cultured live cells.

In general, a well plate may also be referred to as a microplate or a microtiter plate depending on the size of the well and the purpose of use. However, the description "well plate" is used throughout the present specification.

In such in vitro tests, it is known that water evaporates from the culture solution when the cells are cultured for a long period of time. The water evaporation from the culture solution causes fluctuation of the reagent concentration of the culture solution, which affects the cell culture.

Further, it is known that the water evaporation from the culture solution is likely to occur in the wells near the outer periphery of the well plate among the multiple wells provided in a matrix form on the well plate. Therefore, when water evaporates from the culture solution, the concentration of the culture solution is caused to vary between the wells located near the outer periphery of the well plate and the wells located near the center of the well plate, which may cause variations in cell culture.

For addressing such a problem, a well plate having a compartment for holding a liquid for controlling evaporation at a region outside the aligned wells has been proposed (see, for example, Patent Document 1).

DISCLOSURE OF INVENTION

Problems to be Solved by the Invention

However, a well plate generally has a size conforming to the SBS standard. In the well plate described in Patent Document 1, it is necessary to secure a region for forming a required number of wells in the well plate with a size specified by the standard, while forming a compartment for the liquid in the remaining region. Therefore, in the well plate described in Patent Document 1, the amount of the liquid for controlling evaporation that can be stored is limited to a small amount, and it is difficult to suppress the water evaporation from the medium when the cell culture is carried out over a long period of time.

Nowadays, the well plates have been becoming more and more densely integrated, and the volume of culture solution stored per one well has become smaller as compared to the well plates before such dense integration is achieved. This resulted in noticeable adverse effect due to water evaporation from the medium, and countermeasures against this issue have been required.

The present invention has been made in view of such circumstances, and the object of the present invention is to provide a drying suppression device capable of suppressing water evaporation from a medium held by a well plate. Another object of the present invention is to provide a cell culture kit having such a drying suppression device, which enables suitable cell culture.

Means to Solve the Problems

In order to solve the above problems, the present invention, in one aspect thereof, provides a drying suppression device including a main body housing a well plate, and an openable/closable lid provided on the main body, the main body including: a holder section that holds the well plate; a liquid storage tank that stores liquid; and a support section that supports the holder section while keeping the holder section away from an inner bottom surface of the liquid storage tank, wherein, with the lid being closed, an inner space surrounded by the liquid storage tank and the lid communicate with an outside space.

Effect of the Invention

The present invention can provide a drying suppression device capable of suppressing water evaporation from a medium held by a well plate. Further, the present invention can provide a cell culture kit having such a drying suppression device, which enables suitable cell culture.

BRIEF DESCRIPTION OF THE DRAWINGS

FIG. 1 is a schematic perspective view showing a drying suppression device 1 and a cell culture kit 100 of one embodiment.
FIG. 2 is a cross-sectional view as viewed from the arrow direction of the II-II line of FIG. 1.
FIG. 3 is an explanatory diagram of a drying suppression device 1X which does not have the configuration of the present invention, and a cell culture kit 100X having the drying suppression device 1X.
FIG. 4 is an explanatory diagram of a drying suppression device 1 of the embodiment and a cell culture kit 100 having the drying suppression device 1.
FIG. 5 is an explanatory diagram of a drying suppression device 2A of the second embodiment of the present invention, and a cell culture kit 200A having the drying suppression device 2A.
FIG. 6 is an explanatory diagram of a drying suppression device 2B of a modified example of the second embodiment, and a cell culture kit 200B having the drying suppression device 2B.
FIG. 7 is an explanatory diagram of a drying suppression device 3A of the third embodiment of the present invention, and a cell culture kit 300A having the drying suppression device 3A.
FIG. 8 is an explanatory diagram of a drying suppression device 3B of a modified example of the third embodiment, and a cell culture kit 300B having the drying suppression device 3B.
FIG. 9 is an explanatory diagram of a drying suppression device 4 of the fourth embodiment of the present invention, and a cell culture kit 400 having the drying suppression device 4.
FIG. 10 is an explanatory diagram of a drying suppression device 5 of the fifth embodiment of the present invention, and a cell culture kit 500 having the drying suppression device 5.
FIG. 11 is an explanatory diagram of a drying suppression device 6 of the sixth embodiment of the present invention, and a cell culture kit 600 having the drying suppression device 6.
FIG. 12 is an explanatory diagram showing the results of the Examples.
FIG. 13 is an explanatory diagram showing the results of the Examples.
FIG. 14 is an explanatory diagram showing the results of the Examples.
FIG. 15 is an explanatory diagram showing the results of the Examples.

DESCRIPTION OF THE EMBODIMENTS

[First Embodiment]

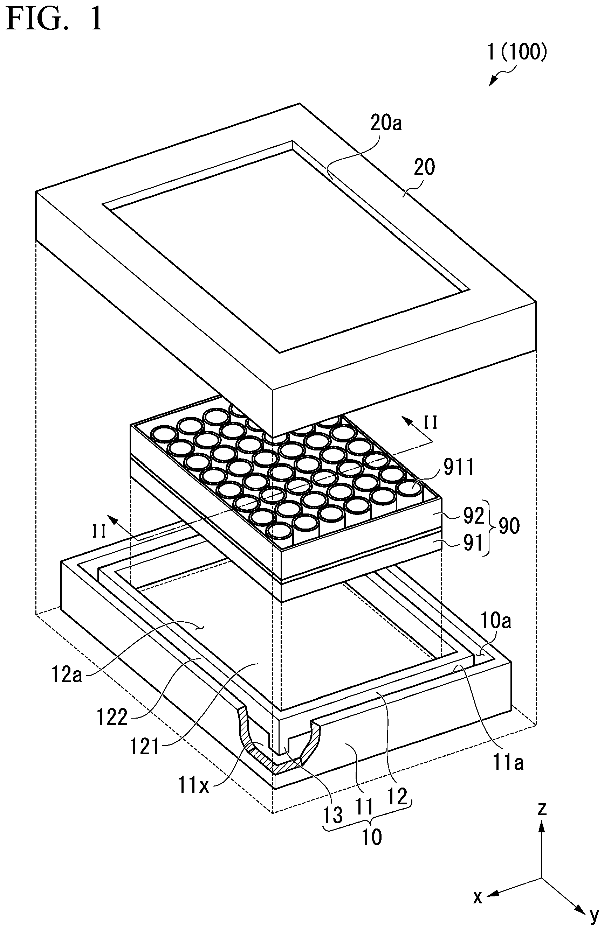

Hereinbelow, the drying suppression device and the cell culture kit according to the first embodiment of the present invention are described with reference to FIGs. 1 to 4. In all the drawings described below, the size and relative dimensions of each component are suitably chosen for facilitating the understanding of the drawings, which hence do not necessarily reflect the actual size and relative dimensions.

Hereinbelow, applying a xyz orthogonal coordinate system to the present invention, some descriptions are made on the positional relationship between the components with reference to the xyz orthogonal coordinate system. In the present specification, a predetermined direction in the horizontal plane is defined as x-axis direction, a direction orthogonal to the x-axis direction in the horizontal plane is defined as y-axis direction, and a direction orthogonal to both of the x-axis direction and the y-axis direction (i.e., vertical direction) is defined as z-axis direction.

Further, for describing the relative positions of the components, a position in the + z axis direction with respect to a certain component may be referred to as "above" or "upper". Likewise, a position in the -z axis direction with respect to a certain component may be referred to as "below" or "lower".

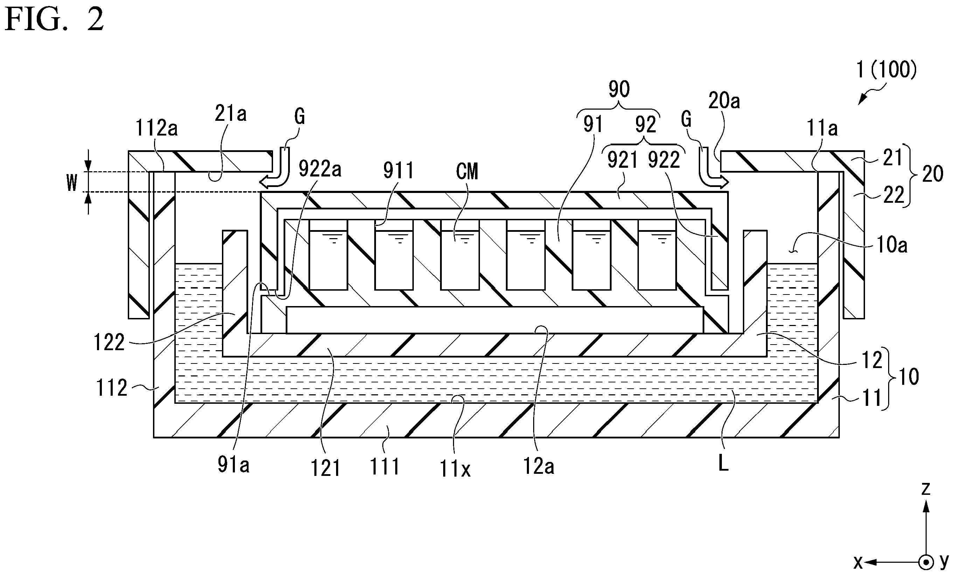

FIG. 1 is a schematic perspective view showing a drying suppression device 1 and a cell culture kit 100 of the present embodiment. FIG. 2 is a cross-sectional view as viewed from the arrow direction of the II-II line of FIG. 1.

<Drying suppression device>

The drying suppression device 1 has a main body 10 and a lid 20. The drying suppression device 1 is used to suppress the evaporation of water from the medium filled in the well plate 90 when culturing the cells accommodated in the well plate 90.

In the context of the present specification, the term "medium" encompasses both a liquid medium (culture medium) and a gel medium. The term "gel medium" means a medium that is used in cell culture, which is a technical field to which the present embodiment belongs, and is semi-solidified to the extent that fluidity is lost. Examples of the "gel medium" include an agar medium and a hydrogel medium.

(Main body)

The main body 10 has a liquid storage tank 11, a holder section 12, and a support section 13.

The liquid storage tank 11 is a container having an opening 11a that opens upward, and houses the holder section 12 and the support section 13 in its inner space. Specifically, the liquid storage tank 11 has a bottom plate 111 and a side wall 112 that is provided on the peripheral edge of the bottom plate 111 and extends upward. The liquid storage tank 11 shown in FIG. 1 is a thin container having a rectangular shape in a plan view.

The liquid storage tank 11 stores the liquid L for suppressing the evaporation of water from the medium held in the well plate 90 in the inner space surrounded by the bottom plate 111 and the side wall 112. The "liquid for suppressing the evaporation of water from the medium" corresponds to the "liquid for suppressing drying" in the present invention.

As the liquid L, a liquid that does not inhibit the growth of cells in the wells 911 is preferable, and pure water is more preferable.

Further, for suppressing the growth of various germs in the liquid L, a preservative or an antibiotic may be added to the liquid L. For example, the liquid L may be an aqueous solution obtained by adding a preservative or an antibiotic to pure water in such an amount as would not inhibit the growth of cells in the wells 911. The amount of the preservative in the liquid L or the amount of the antibiotic in the liquid L may be determined by conducting a preliminary experiment in advance to confirm that the growth of cells in the wells 911 is not inhibited.

The holder section 12 is a component that holds the well plate 90. The holder section 12 has a region 12a for holding the well plate 90. The holder section 12 shown in FIG. 1 is a thin container having a rectangular shape as viewed in plan, and has a bottom plate 121 on which the well plate 90 is mounted, and a side wall 122 that is provided so as to surround the region 12a and extends upward. The region 12a is set to be one size larger than the plan view shape of the well plate 90 conforming to the SBS standard, so that the well plate 90 is allowed to be positioned without interfering with the side wall 122.

The side wall 122 possessed by the holder section 12 enables easy positioning of the well plate to be mounted. Further, when the well plate 90 is mounted on the holder section 12, the well plate 90 is prevented from moving on the holder section 12, whereby handling becomes easy.

The holder section 12 may not have the side wall 122 as long as the region 12a is secured.

Further, as described above, the side wall 122 functions to establish a configuration defining the region 12a, but the configuration defining the region 12a is not limited to this example. When the holder section 12 does not have the side wall 122, a mark such as a recess or a protrusion may be provided on the surface of the bottom plate 121 as a configuration indicating the region 12a.

At the periphery of the region 12a in the holder section 12, the side wall 112 of the liquid storage tank 11 and the side wall 122 of the holder section 12 are separated away from each other as viewed in plan. The gap between the side wall 112 and the side wall 122 functions as a vapor port 10a. The liquid L stored in the liquid storage tank 11 is exposed through the vapor port 10a. The vapor of the liquid L stored in the liquid storage tank 11 is discharged from the liquid storage tank 11 through the vapor port 10a.

The support section 13 supports the holder section 12 by lifting it away from the inner bottom surface 11x of the liquid storage tank 11. The support section 13 shown in FIG. 1 is columnar components provided at the four corners of the holder section 12 as viewed in plan.

The support section 13 is formed integrally with the bottom plate 121 of the holder section 12 at the four corners of the holder section 12. Further, the support section 13 is formed integrally with the liquid storage tank 11. That is, the main body 10 in the present embodiment is a single component in which the liquid storage tank 11, the holder section 12, and the support section 13 are integrally formed.

(Lid)

The lid 20 is a component that is provided openable and closable relative to the main body 10 and covers the opening 11a. The lid 20 is configured to be detachable from the main body 10. Specifically, the lid 20 has a ceiling plate 21 and a side wall 22 that is provided on the peripheral edge of the ceiling plate 21 and extends downward. The lid 20 is fitted with the main body 10 on the inner peripheral side of the side wall 22.

The lower surface 21a of the ceiling plate 21 is in contact with the upper end 112a of the side wall 112 of the liquid storage tank 11, and defines the position of the lid 20 in the height direction. The distance W between the lower surface 21a and the upper surface 90a of the well plate 90 is, for example, preferably 0.1 mm or more and 20 mm or less.

The lid 20 covers the vapor port 10a at least when viewed in plan, and covers the opening 11a with respect to an area ranging from the side wall 112 of the liquid storage tank 11 to the region 12a.

In the present embodiment, the lid 20 is configured to be detachable from the main body 10, but the present invention is not limited to this configuration. For example, the lid 20 may be configured to be connected to the main body 10 via a hinge which makes the lid 20 openable and closable.

The lid 20 has a ventilation hole 20a in the ceiling plate 21. The ventilation hole 20a connects the internal space surrounded by the main body 10 and the lid 20 to the outside atmosphere of the drying suppression device 1. As a result, the drying suppressing device 1 can take in the gas of the outside atmosphere into the internal space through the ventilation hole 20a. Further, the drying suppressing device 1 can discharge the gas from the internal space to the outside through the ventilation hole 20a.

Assuming that the lid 20 shown in FIG. 1 does not have the ventilation hole 20a, the "inner space surrounded by the main body 10 and the lid 20" is a space surrounded by the main body 10 and a hypothetical lid 20 without the ventilation hole 20a.

Further, the "outside space" refers to the outside of a space partitioned by the above-mentioned main body 10 and the hypothetical lid 20 without the ventilation hole 20a.

The ventilation hole 20a is preferably located at a position overlapping with the region 12a of the holder section 12 as viewed in plan. In FIGs. 1 and 2, the ventilation hole 20a is shown as a through hole located at a position overlapping with the vicinity of the center of the region 12a of the ceiling plate 21.

Further, it is preferable that the ventilation hole 20a is not provided with a structure such as a filter that is considered to hinder ventilation. The environment inside the incubator is usually dust-free. Furthermore, since the well plate itself has a lid, contamination with extrinsic factors that affect the results of cell culture is suppressed. Therefore, it is not necessary to provide a filter in the ventilation hole 20a.

In general, it is preferred that the pH of the culture solution in which cells are being cultured falls within the optimum pH range. The optimum pH is, for example, 6.8 to 7.2. A general culture solution is adjusted to have an optimum pH when the $CO_2$ concentration of the environment is 5 to 10 %.

On the other hand, when the culture solution is opened to the air, the culture solution turns alkaline. In addition, the metabolism of the cells being cultured results in excretion of lactic acid into the culture solution, which acidifies the culture solution.

As a countermeasure against such fluctuation of the pH of the culture solution, the carbon dioxide concentration of the ambient atmosphere is usually adjusted to 5 to 10% in an incubator for culturing cells, to thereby adjust the pH of the culture solution. Culturing cells in the well 911 of the well plate 90 necessitates delivery of a gas having its carbon dioxide concentration adjusted to the above value to the culture solution in the well 911. In the following description, "a gas having its carbon dioxide concentration adjusted to the above value" is referred to as "adjusted gas G".

The drying suppression device 1 can take in the adjusted gas G from the surrounding environment through the ventilation hole 20a, and can adjust the culture environment in the cells disposed in the well plate 90.

The drying suppression device 1 is preferably configured to allow inflow of the adjusted gas G into the inner space at an inflow rate of 20 ppm/sec or higher from an ambient atmosphere (surrounding environment) having a $CO_2$ concentration of 5 %. The humidity of the ambient atmosphere is, for example, 90 % (relative humidity at 37 °C).

The inflow rate of the adjusted gas G is more preferably 50 ppm/sec or higher, and even more preferably 100 ppm/sec or higher.

The upper limit of the inflow rate of the adjusted gas G is not particularly limited as long as the cell culture in the well plate is not inhibited, but is preferably substantially 10,000 ppm/sec or lower.

The upper limit value and the lower limit value of the inflow rate of the adjusted gas G can be arbitrarily combined.

The inflow rate of the adjusted gas G is measured by placing NDIR (non-dispersive infrared) type $CO_2$ concentration sensor (HJ-$CO_2$-LOG, manufactured by Sato shoji Corporation) in the holder section of the drying suppression device, while placing the drying suppression device in an atmosphere of $CO_2$ concentration of 5 % and a humidity of 90 % (relative humidity at 37 °C). The increase rate of carbon dioxide concentration per unit time obtained from the measured value of carbon dioxide by the $CO_2$ concentration sensor is defined as the "inflow rate of carbon dioxide".

The inflow rate of the adjusted gas G can be adjusted by controlling the position of the ventilation hole 20a and the opening area of the ventilation hole 20a. The opening area of the ventilation holes 20a can be adjusted by controlling the opening diameter of the ventilation hole 20a and the number of the ventilation hole 20a.

In this context, there is a possibility that the culture conditions of the cells within the well may differ in a strict sense between the wells 911 to which the adjusted gas G taken in from the ventilation hole 20a is easily supplied and the wells 911 to which the adjusted gas G is not easily supplied. On the other hand, as shown in FIGs. 1 and 2, when the ventilation hole 20a is located at a position overlapping with the center of the region 12a, the adjusted gas G taken in through the ventilation hole 20a is easily distributed evenly to a plurality of wells 911 held in in the well plate 90, and it is thereby easy to control the culture conditions.

In the drying suppression device 1 of the present embodiment, the ventilation hole 20a is a through hole formed in the lid 20, but is not limited to this example. The ventilation hole 20a may be provided in at least one of the lid 20 and the liquid storage tank 11 as long as it has a configuration that allows the adjusted gas G in the incubator to be introduced into the drying suppression device 1, and various configurations may be adopted. Further, a tubular structure for introducing the adjusted gas G into the drying suppression device 1 may be provided so as to communicate with the ventilation hole 20a.

The drying suppression device 1 preferably has a residual water content of 75 % or more, which is determined by the following measuring method.

(Measuring method)

An SBS standard 96-well plate with all wells filled with 200 μL each of water is placed in in the main body, followed by leaving the well plate to stand with the liquid storage tank storing water, and with its lid being closed.

A change ratio of water content is determined with respect to an initial value by formula (1) after leaving the well plate to stand for 20 days:

$$\text{(Residual water content) (\%)} = B/A \times 100 \qquad (1)$$

wherein:

A indicates an average value of initial values of volumes of water present in a total of four wells located at the four corners of the well plate; and
B indicates an average value of volumes of water remaining in the four wells located at the four corners of the well plate.

More specifically, all wells of the SBS standard 96-well plate are filled with 200 μL each of water, and then the well plate is covered with a lid. The liquid volumes in the wells at the four corners of the well plate are measured, and an arithmetic mean value of the volumes of water in the wells at the four corners is calculated. The calculated arithmetic mean value is used as an initial value.

Pure water is introduced into and stored in the liquid storage tank, and the well plate covered with the lid is mounted on the holder section of the drying suppression device. The lid of the drying suppression device is closed, and the drying suppression device is kept in an incubator with a temperature of 37 °C and a humidity of 90 % or higher.

20 days later, the liquid volumes in the wells at the four corners of the well plate are measured, and an arithmetic mean value of the liquid volumes in the wells at the four corners is calculated. With respect to the calculated arithmetic mean value, the change ratio of the liquid volume with respect to the initial value is calculated from the above formula (1).

The liquid volumes in the wells are measured using a plate reader. A calibration curve is prepared in advance based on a correlation between the absorbance of the laser beam having a wavelength of 970 nm and the liquid volume in the well, and the liquid volume in the well is calculated from the absorbance of water in each well measured using a plate reader.

The residual water content is preferably 80 % or more, and more preferably 90 % or more. The upper limit of the residual water content is ideally 100 %.

The use of such a drying suppression device 1 can be expected to prevent the outer peripheral region of the well plate from drying. This enables suppression of variation of the culture environment between the wells located at peripheral region of the well plate and the wells located near the center of the well plate, and thereby enables suppression of variation in results of cell culture.

(Well plate)

As the well plate 90, a well plate having a configuration usually used in cell culture, to which is a technical field of the present embodiment belongs, is used. The well plate 90 shown in FIGs. 1 and 2 has a plate main body 91 provided with a plurality of wells 911 and a lid 92 covering the plate main body 91. A well plate 90 having a size conforming to the SBS standard can be used.

Inside the multiple wells 911, cells (not shown) for being cultured and a medium CM used for culturing the cells are accommodated.

The plate main body 91 is provided with, at its peripheral edge region, a step 91a protruding toward the outer periphery thereof.

The lid 92 is a component that is detachably provided on the plate main body 91 and covers a plurality of wells 911 from above. Specifically, the lid 92 has a ceiling plate 921 and a side wall 922 that is provided on the peripheral edge of the ceiling plate 921 and extends downward. The lid 92 is fitted with the plate main body 91 on the inner peripheral side of the side wall 922. The lower end 922a of the side wall 922 is in contact with the step 91a of the plate main body 91, and defines the position of the lid 92 in the height direction.

<Cell culture kit>

The cell culture kit 100 has a drying suppression device 1 and a well plate 90 housed in the drying suppression device 1. The well plate 90 is provided with a plurality of wells 911, and has cells (not shown) housed inside the wells 911 and a medium (not shown) filled inside the wells 911.

For example, the cell culture kit 100 is placed in a known incubator, and is used for culturing the cells contained in the wells 911.

When culturing cells in an incubator extends for a prolonged period of time, the medium in the wells 911 may become dry, such that the culturing conditions for the cells held in the wells 911 deviate from the desired conditions. On the other hand, in the cell culture kit 100 of the present embodiment, the well plate 90 is housed in the above-mentioned drying suppression device 1, whereby the drying of the medium in the wells 911 can be suppressed.

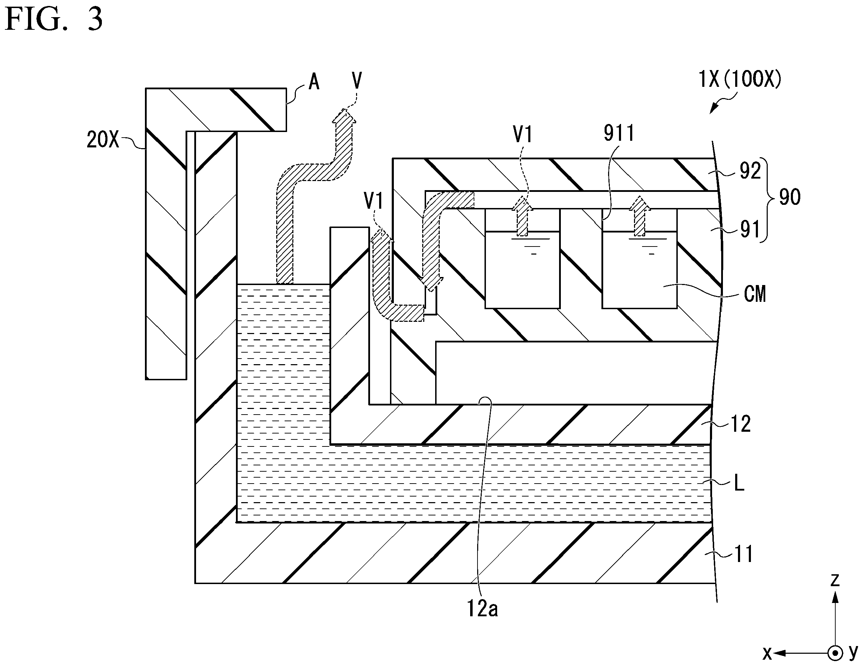

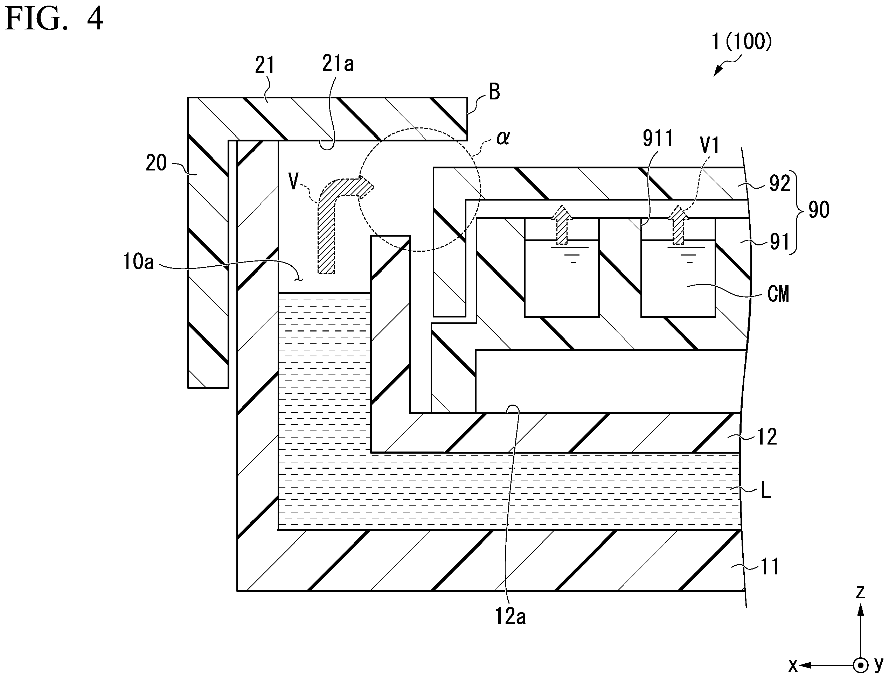

FIGs. 3 and 4 are diagrams for explaining the effects of the drying suppression device of the present embodiment, and are partially enlarged views showing cross sections of peripheral edge regions of the drying suppression device.

FIG. 3 is an explanatory diagram of a drying suppression device 1X which does not have the configuration of the present invention, and a cell culture kit 100X having the drying suppression device 1X. FIG. 4 is an explanatory diagram of a drying suppression device 1 of the present embodiment and a cell culture kit 100 having the drying suppression device 1.

The area covered by the lid differs between the drying suppression device 1 and the drying suppression device 1X.

Specifically, in the lid 20X of the drying suppression device 1X shown in FIG. 3, the end A of the ceiling plate 21X on the ventilation hole side overlaps with the vapor port 10a in a plan view. That is, the lid 20X does not cover the opening 11a with respect to an area ranging from the side wall 112 of the liquid storage tank 11 to the region 12a for holding the well plate 90 in the holder section 12.

On the other hand, in the lid 20 of the drying suppression device 1 shown in FIG. 4, the end B of the ceiling plate 21 on the ventilation hole side overlaps the region 12a in a plan view. That is, as described above, the lid 20 covers the vapor port 10a as viewed in plan, and covers the opening 11a with respect to an area ranging from the side wall 112 of the liquid storage tank 11 to the region 12a for holding the well plate 90 in the holder section 12.

Due to such a difference in configuration, the following difference in effect arises.

First, as shown in FIG. 3, when the well plate 90 is heated, for example, in an incubator (not shown), the evaporation of water from the medium CM in the wells 911 is promoted, and vapor V1 is generated.

In the drying suppression device IX, the evaporation of the liquid L stored in the liquid storage tank 11 is promoted by the heating in an incubator (not shown), and the generated vapor V reaches the lid 20X. In this instance of the drying suppression device IX, since the lid 20X does not cover the opening 11a at an area extending to the region 12a, the vapor V diffuses and spreads further from the ventilation hole of the lid 20X.

Therefore, the peripheral edge region $\alpha$ of the region 12a in a plan view has low humidity. As a result, the water evaporated from the medium CM in the wells 911 is released to the peripheral edge region $\alpha$ through the gap between the plate main body 91 of the well plate 90 and the lid 92, thereby promoting the drying of the medium CM. As the drying of the medium CM proceeds, the cell culture conditions diversify between the wells 911, which may result in difficulty with implementing proper in vitro tests.

On the other hand, also in the drying suppression device 1 shown in FIG. 4, the evaporation of the liquid L stored in the liquid storage tank 11 is promoted by the heating in an incubator (not shown). The generated vapor V forms an air flow directed upward from the vapor port 10a, and reaches the lid 20. In the drying suppression device 1, since the lid 20 covers the opening 11a at an area extending to the region 12a, the air flow of the vapor V spreads along the lower surface 21a of the lid 20 (ceiling plate 21), and fills the peripheral edge region $\alpha$ of the region 12a as viewed in plan.

Since the size of the region 12a is set according to the standardized size of the well plate 90, the peripheral edge region of the well plate 90 overlaps with the peripheral edge region $\alpha$ as viewed in plan. Therefore, in the cell culture kit 100 having the drying suppression device 1, the peripheral edge region of the well plate 90 is filled with vapor V during cell culture. Consequently, the peripheral edge region $\alpha$ of the region 12a as viewed in plan has a higher humidity as compared to the case of the drying suppression device 1X.

In the well plate 90 disposed in the drying suppression device 1 having such a configuration, the water evaporated from the medium CM in the wells 911 can hardly diffuse toward the outside of the well plate 90, and the drying of the medium CM is suppressed. As a result, the difference in cell culture conditions between the wells 911 is unlikely to occur, and favorable cell culture is realized, thereby enabling proper in vitro tests.

Further, in the drying suppression device 1, the holder section 12 is lifted by the support section 13, and a space is formed below the holder section 12 where the liquid L can be stored. Therefore, the drying suppression device 1 can store a larger volume of the liquid L in the liquid storage tank 11 as compared to a drying suppression device with a configuration wherein the holder section 12 is provided directly on the bottom surface of the liquid storage tank 11 and no space is formed below the holding unit 12, and such a drying suppression device 1 can suppress the evaporation of water from the medium CM held in the wells 911 over a longer period of time.

The drying suppression device 1 having the above configuration can suppress the evaporation of water from the medium CM held by the well plate 90.

Further, the cell culture kit 100 having the above-described configuration has the above-mentioned drying suppression device 1 and enables suitable cell culture.

[Second Embodiment]

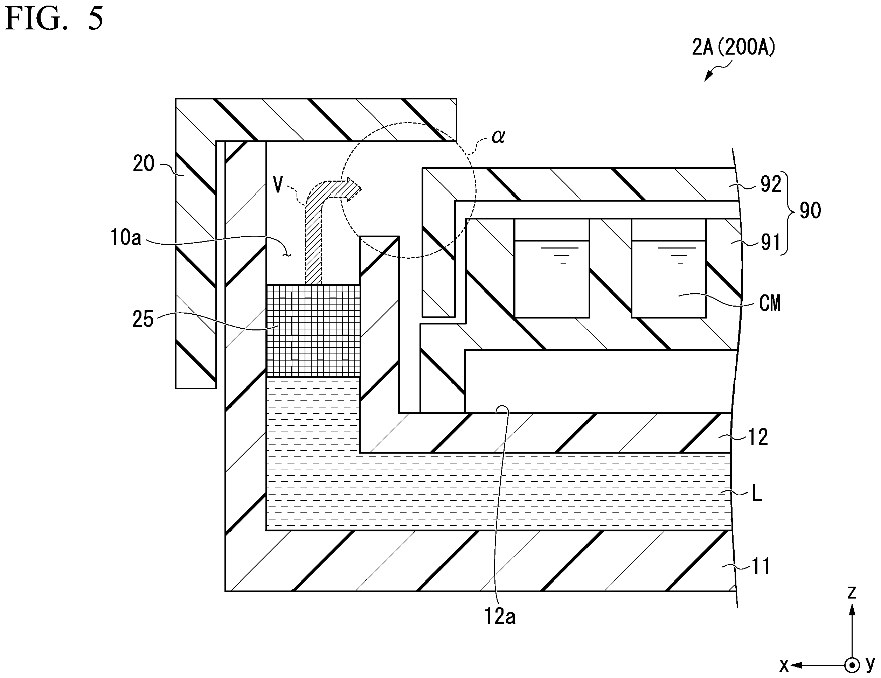

FIG. 5 is an explanatory diagram of a drying suppression device 2A of the second embodiment of the present invention, and a cell culture kit 200A having the drying suppression device 2A. In the following embodiments, the components already mentioned are designated by the same reference numerals, and detailed description thereof will be omitted.

In the drying suppression device 2A shown in FIG. 5, a porous member 25 is provided at the vapor port 10a. The porous member 25 is placed so as to close the vapor port 10a.

The porous member 25 has, as a whole, a function of sucking up the liquid L through the capillary phenomenon. Examples of the material for forming the porous member 25 include a spongy material having a plurality of pores which communicate mutually within the material.

The material for forming the porous member 25 may be an organic substance such as a non-woven fabric, felt, a woven fabric or a sponge, or an inorganic substance such as pumice stone or porous ceramics. The sponge may be a natural one or a synthetic one.

The cell culture kit 200A has the above-mentioned drying suppression device 2A and a well plate 90.

In the drying suppression device 2A, since the porous member 25 closes the vapor port 10a, the liquid L is less likely to spill when the cell culture kit 200A is being transferred, and the handling becomes easier.

Further, when using the drying suppression device 2A, for example, the volume of the liquid L may be adjusted so that the porous member 25 is in contact with the liquid L in the liquid storage tank 11 while preventing the porous member 25 from being completely immersed in the liquid L. The porous member 25 in contact with the liquid L sucks up the liquid L and promotes the evaporation of the liquid L. As a result, in the cell culture kit 200A having the drying suppression device 2A, the peripheral edge region of the well plate 90 (peripheral edge region $\alpha$ of the region 12a) is filled with vapor V during cell culture. Consequently, in the cell culture kit 200A, the water evaporation from the medium CM in the wells 911 is suppressed.

Alternatively, when the drying suppressing device 2A is being used, the liquid level of the liquid L may be positioned below the porous member 25 without the porous member 25 coming into contact with the liquid L in the liquid storage tank 11. Even in this instance, the vapor V generated by the evaporation of the liquid L is supplied to the peripheral edge region $\alpha$ through the pores of the porous member 25 and fills the peripheral edge region $\alpha$. Consequently, in the cell culture kit 200A, the water evaporation from the medium CM in the wells 911 is suppressed.

In addition to the above-mentioned effect, the drying suppression device 2A having the above configuration can also suppress the evaporation of water from the medium CM held by the well plate 90.

Further, the cell culture kit 200A having the above-described configuration also has the above-mentioned drying suppression device 2A and enables suitable cell culture.

(Modified example)

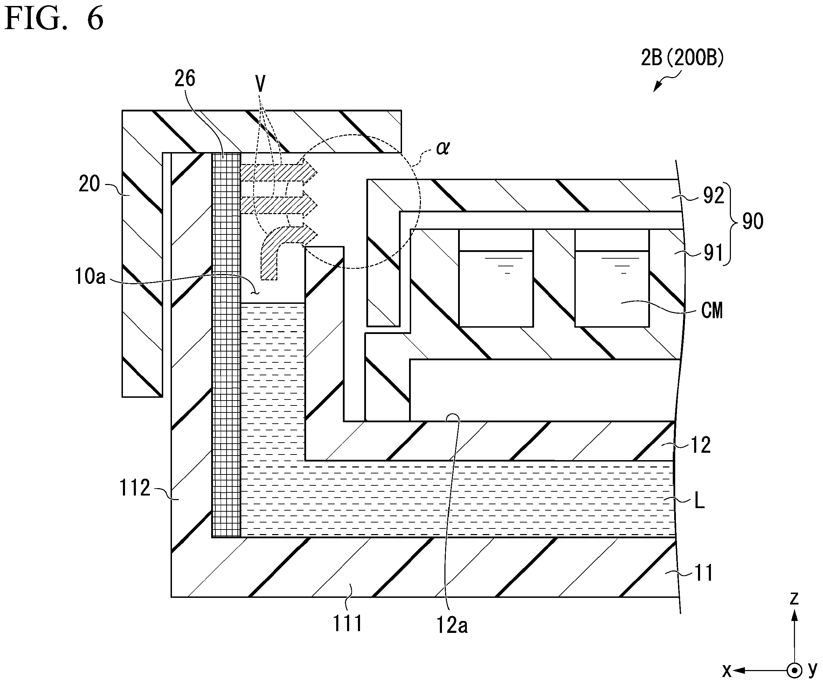

FIG. 6 is an explanatory diagram of a drying suppression device 2B of a modified example of the present embodiment, and a cell culture kit 200B having the drying suppression device 2B. FIG. 6 corresponds to FIG. 5.

As shown in FIG. 6, the porous member 26 included in the drying suppression device 2B is provided at the vapor port 10a, and the lower end thereof is in contact with the bottom surface 11x of the liquid storage tank 11. As a result, the porous member 26 is always in contact with the liquid L regardless of the volume of the liquid L stored in the liquid storage tank 11, and the evaporation of the liquid L can be promoted.

Further, as shown in FIG. 6, the porous member 26 may extend above the holder section 12. The maximum volume of the liquid L that can be stored in the liquid storage tank 11 is the volume at which the liquid level of the liquid L is at the same height as the upper end of the holder section 12. Therefore, when the porous member 26 extends above the holder section 12, the liquid L can be sucked up above the liquid level of the liquid L when the porous member 26 is absent, and the evaporation of the liquid L can thereby be promoted.

In addition to the above-mentioned effect, the drying suppression device 2B having the above configuration can also suppress the evaporation of water from the medium CM held by the well plate 90.

Further, the cell culture kit 200B having the above-described configuration also has the above-mentioned drying suppression device 2 and enables suitable cell culture.

[Third Embodiment]

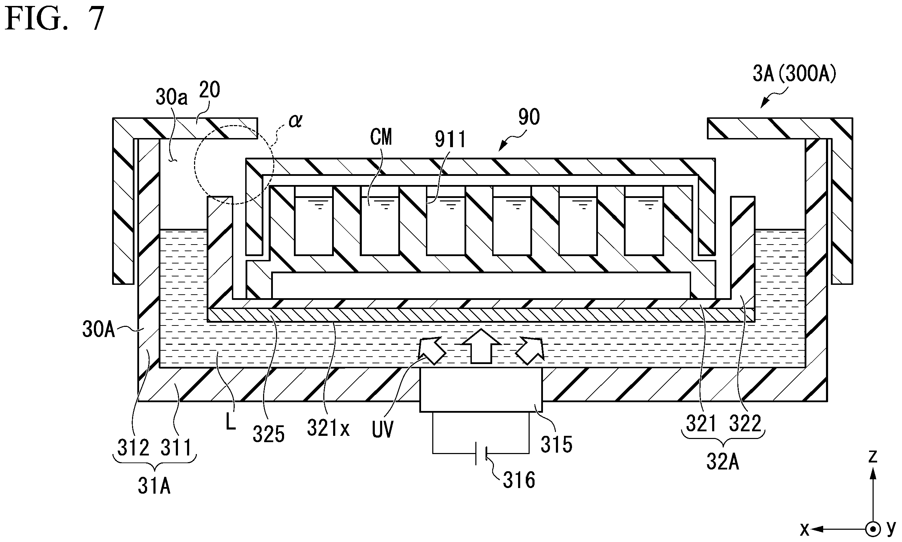

FIG. 7 is an explanatory diagram of a drying suppression device 3A of the third embodiment of the present invention, and a cell culture kit 300A having the drying suppression device 3A. FIG. 7 corresponds to FIG. 2.

The drying suppression device 3A shown in FIG. 7 has a main body 30A and a lid 20. The main body 30A has a liquid storage tank 31A, a holder section 32A, and a support section (not shown).

The liquid storage tank 31A has a bottom plate 311 and a side wall 312 that is provided on the peripheral edge of the bottom plate 311 and extends upward. The bottom plate 311 has a light source 315 that irradiates ultraviolet rays UV toward the inside of the liquid storage tank 31. The light source 315 is connected to the external power supply 316.

As the light source 315, any known light source capable of emitting ultraviolet rays UV can be used. As the light source 315, for example, a light emitting diode (UV-LED) that emits ultraviolet rays can be used.

The holder section 32A has a bottom plate 321 and a side wall 322 that is provided on the peripheral edge of the bottom plate 321 and extends upward. The bottom plate 321 has a light shielding means 325 at a position opposite to the light source 315.

The light-shielding means 325 absorbs and attenuates ultraviolet rays UV. The light-shielding means 325 is, for example, a black layer provided on the outer bottom surface 321x of the bottom plate 321. The material for forming the light-shielding means 325 is a material having an absorption band at the wavelength of ultraviolet rays UV emitted from the light source 315. The light-shielding means 325 may be formed by applying a black paint, or may be formed by laminating a film or a plate on the bottom surface 321x. Further, by forming the holder section 32 using a black-colored resin material, the function of the light-shielding means 325 may be imparted to the entire holder section 32A.

In the above example, the color of the light-shielding means 325 is black, but is not limited to this color in the present invention. As the color of the light-shielding means 325, any of various colors capable of attenuating ultraviolet rays UV can be adopted.

Further, the light-shielding means 325 may also be provided on either one of or both of the surface of the side wall 312 facing the liquid L stored in the liquid storage tank 31A and the surface of the side wall 322 of the holder section 32A facing the liquid L.

The cell culture kit 300A has the above-mentioned drying suppression device 3A and a well plate 90.

In the drying suppression device 3A having such a configuration, the gap between the side wall 312 and the side wall 322 functions as a vapor port 30a. In the drying suppression device 3A, the vapor V generated by the evaporation of the liquid L is supplied to the peripheral edge region α via the vapor port 30a. As a result, in the well plate 90 placed in the drying suppression device 3A, the water evaporated from the medium CM in the wells 911 can hardly diffuse toward the outside of the well plate 90, and the drying of the medium CM is suppressed.

Further, in the drying suppression device 3A, the irradiation of ultraviolet rays UV from the light source 315 kills germs capable of propagating in the liquid L, and can thereby suppress the propagation of germs. After passing through the liquid L, the ultraviolet rays UV reach the light-shielding means 325 and are attenuated. Therefore, the ultraviolet rays UV do not reach the well plate 90 and do not inhibit the cell culture performed in the well plate 90.

The drying suppression device 3A having the above configuration can also suppress the evaporation of water from the medium CM held by the well plate 90.

Further, the cell culture kit 300A having the above configuration also has the above-mentioned drying suppression device 3A and enables suitable cell culture.

(Modified example)

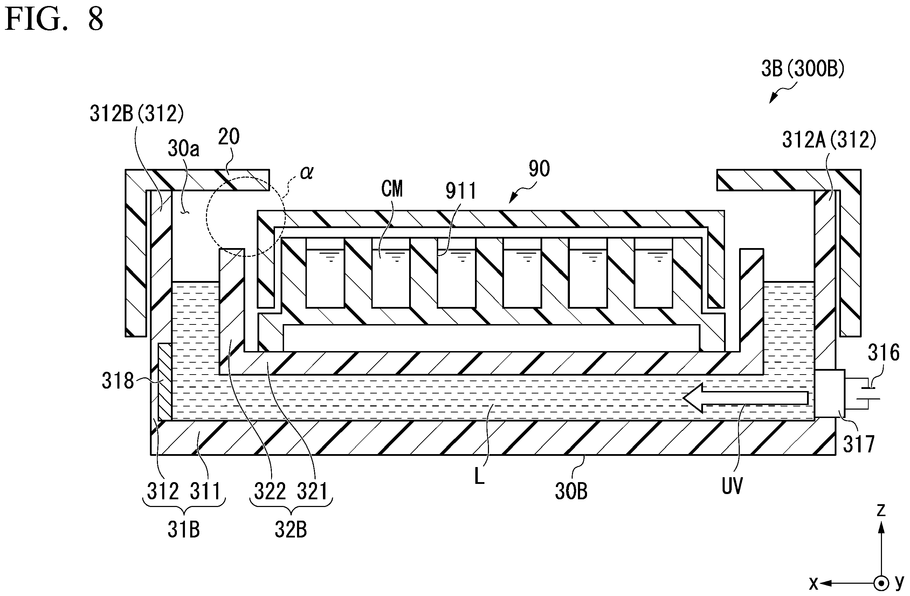

FIG. 8 is an explanatory diagram of a drying suppression device 3B of a modified example of the present embodiment, and a cell culture kit 300B having the drying suppression device 3B. FIG. 8 corresponds to FIG. 5.

As shown in FIG. 8, the drying suppression 3B has a main body portion 30B and a lid 20. The main body 30B has a liquid storage tank 31B, a holder section 32B, and a support section (not shown).

The liquid storage tank 31B has a bottom plate 311 and a side wall 312. The side wall 312A has a light source 317 that irradiates ultraviolet rays UV toward the inside of the liquid storage tank 31. The light source 317 is connected to the external power supply 316. As the light source 317, a light source having the same configuration as the above-mentioned light source 315 can be adopted.

Further, the liquid storage tank 31B has a light-shielding means 318 on the side wall 312B facing the side wall 312A provided with the light source 317. The light-shielding means 318 can be configured to have the same properties by using the same material as the above-mentioned light-shielding means 325.

Further, the light-shielding means 318 may also be provided on either one of or both of the surface of the side wall 312 facing the liquid L stored in the liquid storage tank 31B and the surface of the side wall 322 of the holder section 32B facing the liquid

L.

The cell culture kit 300B has the above-mentioned drying suppression device 3B and a well plate 90.

In the drying suppression device 3B with such a configuration, the irradiation of ultraviolet rays UV from the light source 317 kills germs capable of propagating in the liquid L, and can thereby suppress the propagation of germs. After passing through the liquid L, the ultraviolet rays UV reach the light-shielding means 318 and are attenuated. Therefore, the ultraviolet rays UV do not reach the well plate 90 and do not inhibit the cell culture performed in the well plate 90.

In addition to the above-mentioned effect, the drying suppression device 3B having the above configuration can also suppress the evaporation of water from the medium CM held by the well plate 90.

Further, the cell culture kit 300B having the above configuration also has the above-mentioned drying suppression device 3B and enables suitable cell culture.

[Fourth Embodiment]

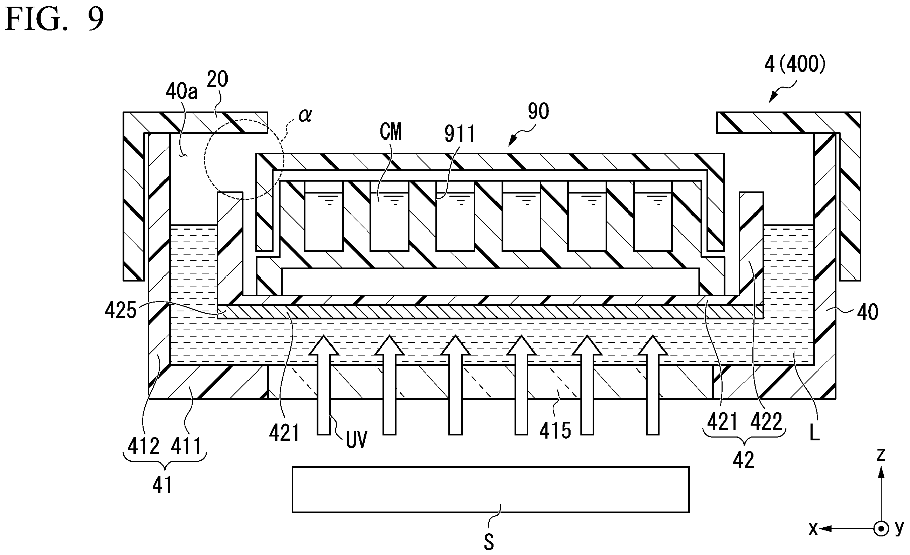

FIG. 9 is an explanatory diagram of a drying suppression device 4 of the fourth embodiment of the present invention, and a cell culture kit 400 having the drying suppression device 4. FIG. 9 corresponds to FIG. 2.

The drying suppression device 4 shown in FIG. 9 has a main body 40 and a lid 20. The main body 40 has a liquid storage tank 41, a holder section 42, and a support section (not shown).

The liquid storage tank 41 has a bottom plate 411 and a side wall 412 that is provided on the peripheral edge of the bottom plate 411 and extends upward. The bottom plate 411 has a light-collecting window 415 formed of a ultraviolet light-transmissive material.

The light-collecting window 415 transmits ultraviolet rays UV emitted from the external light source S and leads ultraviolet rays UV into the inside of the liquid storage tank 41. The material for forming the light-collecting window 415 is a material having no absorption band at the wavelength of the ultraviolet rays UV. Examples of the material for forming the light-collecting window 415 include quartz glass.

The holder section 42 has a bottom plate 421 and a side wall 422 that is provided on the peripheral edge of the bottom plate 421 and extends upward. The bottom plate 421 has a light-shielding means 425 at a position opposite to the light-collecting window 415. The light-shielding means 425 can be configured to have the same properties by using the same material as the above-mentioned light-shielding means 325.

Further, the light-shielding means 425 may also be provided on either one of or both of the surface of the side wall 412 facing the liquid L stored in the liquid storage tank 41 and the surface of the side wall 422 of the holder section 42 facing the liquid L.

The cell culture kit 400 has the above-mentioned drying suppression device 4 and a well plate 90.

In the drying suppression device 4 having such a configuration, the gap between the side wall 412 and the side wall 422 functions as a vapor port 40a. In the drying suppression device 4, the vapor V generated by the evaporation of the liquid L is supplied to the peripheral edge region $\alpha$ via the vapor port 40a. As a result, in the well plate 90 disposed in the drying suppression device 4, the water evaporated from the medium CM in the well 911 can hardly diffuse toward the outside of the well plate 90, and the drying of the medium CM is suppressed.

Further, in the drying suppression device 4, the ultraviolet rays UV irradiated to the inside of the liquid storage tank 41 through the light-collecting window 415 can kill germs capable of propagating in the liquid L, and can thereby suppress the propagation of germs. After passing through the liquid L, the ultraviolet rays UV reach the light-shielding means 425 and are attenuated. Therefore, the ultraviolet rays UV do not reach the well plate 90 and do not inhibit the cell culture performed in the well plate 90.

The drying suppression device 4 having the above configuration can also suppress the evaporation of water from the medium CM held by the well plate 90.

Further, the cell culture kit 400 having the above-described configuration also has the above-mentioned drying suppression device 4 and enables suitable cell culture.

[Fifth Embodiment]

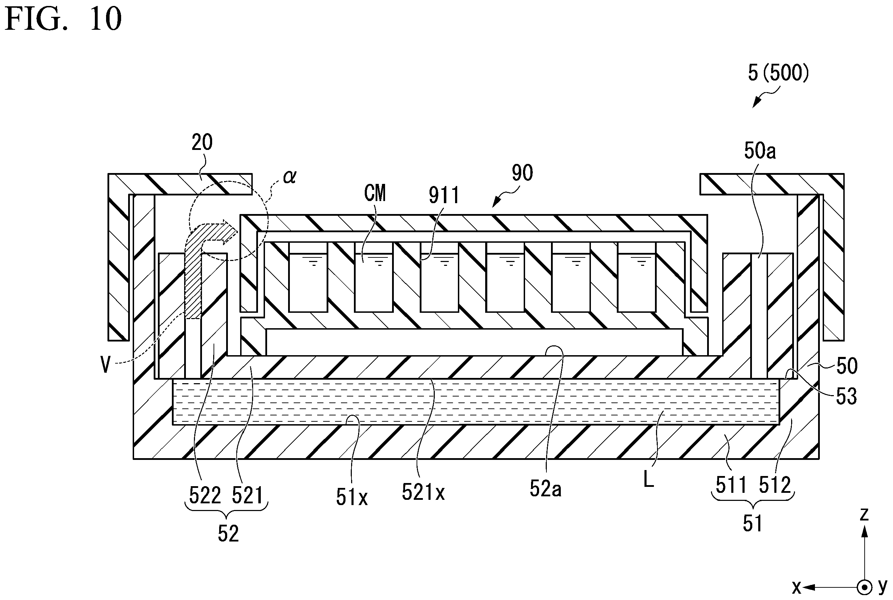

FIG. 10 is an explanatory diagram of a drying suppression device 5 of the fifth embodiment of the present invention, and a cell culture kit 500 having the drying suppression device 5. FIG. 10 corresponds to FIG. 2.

The drying suppression device 5 shown in FIG. 10 has a main body 50 and a lid 20. The main body 50 has a liquid storage tank 51 and a holder section 52.

The liquid storage tank 51 has a bottom plate 511 and a side wall 512 that is provided on the peripheral edge of the bottom plate 511 and extends upward. The inner peripheral surface of the side wall 512 is provided with a step protruding toward the inside of the liquid storage tank 51. The step functions as the support section in the present embodiment. In the following description, the step provided on the side wall 512 is referred to as a support section 53.

The support section 53 may be provided on the inner peripheral surface of the side wall 512 in a closed annular shape without terminal end as viewed in plan, or may be provided discretely at multiple locations on the inner peripheral surface.

The holder section 52 is provided so as to be separable from the liquid storage tank 51, and covers the inner bottom surface 51x of the liquid storage tank 51. The holder section 52 has a bottom plate 521 and a side wall 522 that is provided on the peripheral edge of the bottom plate 521 and extends upward. The side wall 522 surrounds the region 52a that holds the well plate 90 in the holder section 52.

The holder section 52 is in contact with and supported by the support section 53 at the peripheral edge region of the outer bottom surface 521x of the bottom plate 521. The liquid L is stored in a space surrounded by the liquid storage tank 51 and the holder section 52.

The side wall 522 of the holder section 52 is provided with a through hole that penetrates the holder section 52 in the thickness direction of the holder section 52. The through hole functions as a vapor port in this embodiment. In the following description, the through hole provided in the holder section 52 is referred to as a vapor port 50a. The lower end of the vapor port 50a is open to the space where the liquid L is stored.

The cell culture kit 500 has the above-mentioned drying suppression device 5 and a well plate 90.

In the drying suppression device 5, the vapor V generated by the evaporation of the liquid L is supplied to the peripheral edge region α via the vapor port 50a. As a result, in the well plate 90 disposed in the drying suppression device 5, the water evaporated from the medium CM in the wells 911 can hardly diffuse toward the outside of the well plate 90, and the drying of the medium CM is suppressed.

In addition to the above-mentioned effect, the drying suppression device 5 having the above configuration can also suppress the evaporation of water from the medium CM held by the well plate 90.

Further, the cell culture kit 500 having the above-described configuration also has the above-mentioned drying suppression device 5 and enables suitable cell culture.

[Sixth Embodiment]

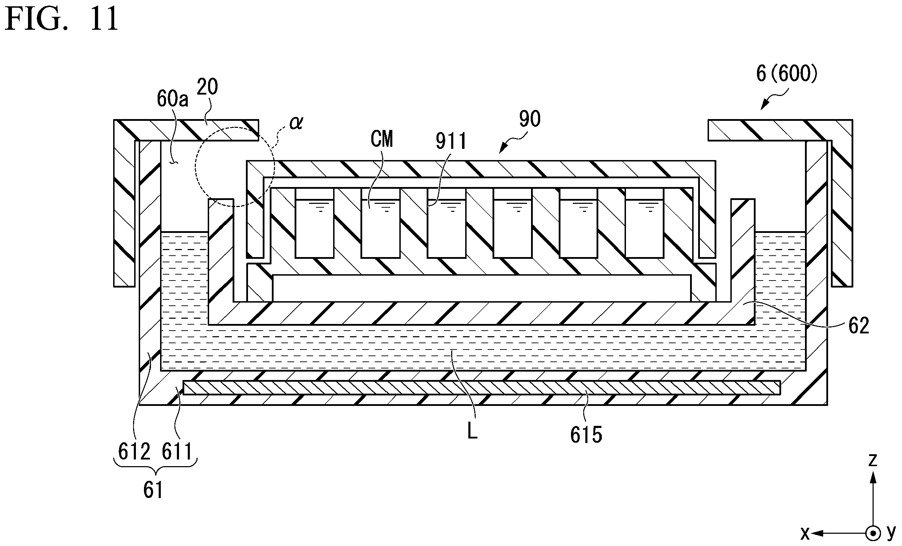

FIG. 11 is an explanatory diagram of a drying suppression device 6 of the sixth embodiment of the present invention, and a cell culture kit 600 having the drying suppression device 6. FIG. 11 corresponds to FIG. 2.

The drying suppression device 6 shown in FIG. 11 has a main body 60 and a lid 20. The main body 60 has a liquid storage tank 61, a holder section 62, and a support section (not shown).

The liquid storage tank 61 has a bottom plate 611 and a side wall 612 that is provided on the peripheral edge of the bottom plate 611 and extends upward. The bottom plate 611 has a heating unit 615. The heating unit 615 may be embedded in the bottom plate 611 or may be provided on and in contact with the surface of the bottom plate 611.

The holder section 62 may have the same configuration as the holder section 12 described above.

The cell culture kit 600 has the above-mentioned drying suppression device 6 and a well plate 90.

In the drying suppression device 6, the heating unit 615 heats the liquid L indirectly by heating the liquid storage tank 61 or directly by contacting the liquid L. As a result, when the cell culture kit 600 is taken out from the incubator, the drying suppression device 6 can be heated and the well plate 90 can be kept warm.

Therefore, when the drying suppression device 6 is used, even if there is a temperature difference between the inside and outside of the incubator, the influence of the temperature change on the cells cultured by the cell culture kit 600 can be reduced, to thereby stabilize the cell culture conditions.

Further, the gap between the side wall 612 and the holder section 62 functions as a vapor port 60a. In the drying suppression device 6, the vapor V generated by the evaporation of the liquid L is supplied to the peripheral edge region α via the vapor port 60a. As a result, in the well plate 90 disposed in the drying suppression device 6, the water evaporated from the medium CM in the wells 911 can hardly diffuse toward the outside of the well plate 90, and the drying of the medium CM is suppressed.

The drying suppression device 6 having the above configuration can also suppress the evaporation of water from the medium CM held by the well plate 90. Further, the cell culture kit 600 having the above-described configuration also has the above-mentioned drying suppression device 6 and enables suitable cell culture.

The preferred embodiments of the present invention are as described above with reference to the accompanying drawings; however, the present invention is not limited to such embodiments. The various shapes and combinations of the respective components shown in the above examples are mere examples, and various alterations can be made depending on design requirements and the like as long as such alterations do not deviate from the gist of the present invention.

[Examples]

Hereinbelow, the present invention will be described in more detail with reference to the Examples which, however, should not be construed as limiting the present invention.

[Evaluation of change in liquid volume]

(Example 1)

All wells of the SBS standard 96-well plate were filled with 200 $\mu$L each of water, and then the well plate was covered with a lid. The liquid volumes in the wells at the four corners of the well plate were measured, and the arithmetic mean value of the volumes of water in the wells at the four corners was calculated. The calculated arithmetic mean value was used as an initial value.

A cell culture kit having the same shape as the cell culture kit 100 shown in FIGs. 1 and 2 was prepared, and pure water was introduced into and stored in the liquid storage tank. The covered well plate was mounted on the holder section of the cell culture kit, covered with the lid, and then held in an incubator having a temperature of 37 °C and a humidity of 90 % or higher.

After the lapse of a predetermined period of time, the liquid volumes in the wells at the four corners of the well plate were measured, and the arithmetic mean value of the liquid volumes in the wells at the four corners was calculated. Further, with respect to the calculated arithmetic mean value, the change ratio of the liquid volume with respect to the initial value was calculated.

The liquid volumes in the wells were measured using a plate reader. A calibration curve was prepared in advance based on a correlation between the absorbance of the laser beam having a wavelength of 970 nm and the liquid volume in the well, and the liquid volume in the well was calculated from the absorbance at each well measured using a plate reader.

(Comparative Example 1)

The same procedures as in Example 1 were repeated except that the cell culture kit was not used, to thereby determine the change ratio of the liquid volume in the wells.

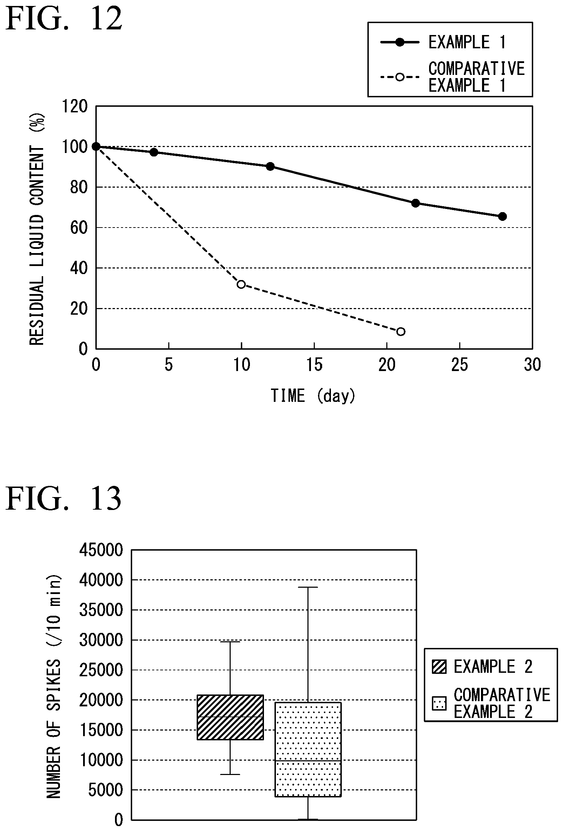

FIG. 12 is a graph showing the change ratios of the liquid volume in Example 1 and Comparative Example 1. As shown in FIG. 12, it was found that the use of the cell culture kit enabled prevention of drying at the four corners of the well plate. This result demonstrate that the use of the cell culture kit can be expected to prevent the outer periphery of the well plate from drying.

[Evaluation of effects on cell culture]

(Example 2)

Nerve cells were seeded in all wells of a 48-well plate of MEA (Micro Electrode Array) manufactured by Axion Biosystems. As the nerve cells, those differentiated from iPS-derived healthy strains into nerve cells were used. Further, the nerve cells were mixed with primary astrocytes and then seeded in the wells. As the astrocytes, Human Primary Astrocyte (Thermo Fisher Scientific Inc.) was used.

A cell culture kit having the same shape as the cell culture kit 100 shown in FIGs. 1 and 2 was prepared, and pure water was introduced into and stored in the liquid storage tank. The covered well plate was mounted on the holder section of the cell culture kit, covered with the lid, and then held in an incubator having a temperature of 37 °C and a humidity of 90 % or higher.

After culturing the nerve cells for 4 weeks, the action potential of the nerve cells was measured using Meastro manufactured by Axion Biosystems. The number of spontaneous firings (spikes) was measured for 10 minutes in all wells, and the variation in the number of spontaneous firings for the wells was determined.

Next, the number of spontaneous firings was measured for 10 minutes when a reagent was added to the cultured nerve cells. As the reagent, a solution of picrotoxin which is a GABA antagonist in dimethyl sulfoxide (DMSO) was used.

First, 300 $\mu$L of 1 % by volume aqueous DMSO solution was added to each of the wells (6 wells) in one row, and the number of spontaneous firings was measured for 10 minutes.

Then, the DMSO solution of picrotoxin was added stepwise to the same wells until the picrotoxin concentration in the wells became 0.1 $\mu$mol/L, 1 $\mu$mol/L, and 10 $\mu$mol/L, and the number of spontaneous firings at each picrotoxin concentration was measured for 10 minutes. At each picrotoxin concentration, the variation in the number of 10-minute spontaneous firings was determined.

(Comparative Example 2)

The variation in the number of spontaneous firings of nerve cells for the wells was determined in the same manner as in Example 2 except that the cell culture kit was not used.

FIG. 13 is a diagram showing variations in the number of spontaneous firings of nerve cells for the wells with respect to the cultured nerve cells of Example 2 and Comparative Example 2. As shown in FIG. 13, the variation in Example 2 using the cell culture kit was smaller than that in Comparative Example 2, which shows that the variation in maturation of the cultured cells can be suppressed by using the cell culture kit.

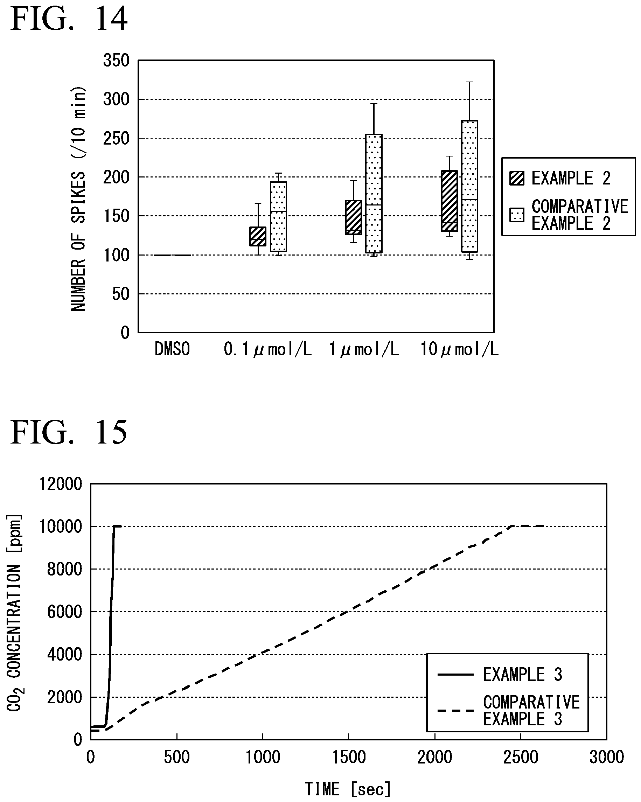

FIG. 14 is a diagram showing variations in response of the nerve cells to the reagent with respect to the cultured nerve cells of Example 2 and Comparative Example 2. As shown in FIG. 14, it was found that the variation in response in Example 2 using the cell culture kit was smaller than that in Comparative Example 2, which shows that the variation in response of the cultured cells can be suppressed by using the cell culture kit.

[Evaluation of carbon dioxide inflow rate]

(Example 3)

The drying suppression device shown in FIG. 1 was used. The size of the ventilation hole 20a in a plan view was 79 mm $\times$ 106 mm. The size of the region 12a in a plan view was 82 mm $\times$ 124 mm. With respect to the lid 20 of the drying suppression device used, the end thereof on the ventilation hole side was overlapping with the region 12a in a plan view.

NDIR (non-dispersive infrared) type $CO_2$ concentration sensor (HJ-CO$_2$-LOG, manufactured by Sato shoji Corporation) was placed in the holder section of the drying suppression device, while placing the drying suppression device in an atmosphere of $CO_2$ concentration of 5 % and a humidity of 90 % (relative humidity at 37 °C). The increase rate of carbon dioxide concentration per unit time obtained from the measured value of carbon dioxide by the $CO_2$ concentration sensor was determined as the "inflow rate of carbon dioxide".

(Comparative Example 3)

The inflow rate of carbon dioxide was determined in the same manner as in Example 3 except that a commercially available drying suppression device (Olaf evaporation prevention chamber, manufactured by Nippon Genetics Co., Ltd.) was used.

The commercially available product used had a configuration in which a well plate-mounted space is sealed in order to suppress the evaporation of water placed in the well plate. However, the commercially available product used did not have a liquid storage tank for storing the liquid for suppressing drying, and does not satisfy the requirement of the present invention.

In addition, the commercially available product used had a ventilation hole on the side wall of the main body housing the well plate. The ventilation hole was provided with a filter covering the ventilation hole.

The results of measurements are shown in FIG. 15. The results of measurements show that the inflow rate of carbon dioxide in the drying suppression device used in Example 3 was 180 ppm/sec. On the other hand, the inflow rate of carbon dioxide in the commercially available product used in Comparative Example 3 was 4 ppm/sec.

When the drying suppression device of the present invention (Example 3) is used, the inflow rate of carbon dioxide is 20 ppm/sec or more, and it is easy to control and equalize the culture conditions inside the drying suppression device.

On the other hand, in the case of the drying suppression device of Comparative Example 3, drying can be suppressed by sealing the well plate; however, it was found that the inflow rate of carbon dioxide is small and it is difficult to control and equalize the culture conditions inside the drying suppression device.

The present invention includes the following embodiments.

[1] A drying suppression device including: a main body housing a well plate; and an openable/closable lid provided on the main body, the main body including: a holder section that holds the well plate; a liquid storage tank that stores liquid; and a support section that supports the holder while keeping the holder section away from an inner bottom surface of the liquid storage tank, wherein, with the lid being closed, an inner space surrounded by the liquid storage tank and the lid communicate with an outside space.

[2] The drying suppression device according to [1], wherein at least one of the lid and the liquid storage tank has a ventilation hole connecting the inner space and the outside space.

[3] The drying suppression device according to [2], wherein the liquid storage tank has an opening that opens above the holder section, the lid covers the opening, and the ventilation hole is provided in the lid.

[4] The drying suppression device according to [3], which has a vapor port provided around the holder section as viewed in plan, through which a vapor of the liquid stored in the liquid storage tank is allowed to flow, wherein: the lid overlaps with the vapor port as viewed in plan, and the ventilation hole overlaps with a region where the well plate is held by the holder section as viewed in plan, while not overlapping with the vapor port as viewed in plan.

[5] The drying suppression device according to any one of [1] to [4], wherein the holder section includes: a bottom plate on which the well plate is mounted; and a side wall provided on the bottom plate so as to surround a region holding the well plate.

[6] The drying suppression device according to any one of [1] to [5], which has a vapor port provided around the holder section as viewed in plan, through which a vapor of the liquid stored in the liquid storage tank is allowed to flow, wherein the vapor port is provided with a porous member.

[7] The drying suppression device according to [6], wherein the porous member closes the vapor port.

[8] The drying suppression device according to [6] or [7], wherein the porous member extends above the holder section.

[9] The drying suppression device according to any one of [6] to [8], wherein the porous member extends to the bottom surface.

[10] The drying suppression device according to any one of [1] to [9], wherein the main body has a light source that emits ultraviolet rays toward the inside, and a light-shielding means provided so as to face the light source.

[11] The drying suppression device according to [10], wherein the light source is provided on the bottom plate of the liquid storage tank, and the light-shielding means is provided on the bottom surface located outside the holder section.

[12] The drying suppression device according to [10] or [11], wherein the light source is provided on a side wall of the liquid storage tank, and the light-shielding means is provided on the side wall of the liquid storage tank at a position opposite to a position where the light source is provided.

[13] The drying suppression device according to any one of [1] to [9], wherein the bottom plate of the liquid storage tank has a light-collecting window formed of a ultraviolet light-transmissive material, and the holder section has a light-shielding means at a position opposite to the light-collecting window.

[14] The drying suppression device according to any one of [1] to [13], wherein the support section of the main body is separable from the liquid storage tank and the holder section.

[15] The drying suppression device according to any one of [1] to [14], which has a vapor port provided around the holder section as viewed in plan, through which a vapor of the liquid stored in the liquid storage tank is allowed to flow, wherein the holder section is positioned away from the side wall of the liquid storage tank as viewed in plan, and the vapor port is a gap between the holder section and the side wall.

[16] The drying suppression device according to any one of [1] to [15], which has a vapor port provided around the holder section as viewed in plan, through which a vapor of the liquid stored in the liquid storage tank is allowed to flow, wherein: the vapor port is a through hole that penetrates the holder section in a thickness direction thereof; and the through hole is provided outside a region of the holder section where the well plate is held.

[17] The drying suppression device according to any one of [1] to [16], wherein the liquid storage tank has a heating unit.

[18] The drying suppression device according to any one of [1] to [17], which is configured to allow inflow of $CO_2$ into the inner space at an inflow rate of 20 ppm/second or higher from an ambient atmosphere having a $CO_2$ concentration of 5 %.

[19] A drying suppression device comprising a main body housing a well plate, and an openable/closable lid provided on the main body, the main body comprising: a holder section that holds the well plate; a liquid storage tank that stores liquid; and a support section that supports the holder while keeping the holder section away from an inner bottom surface of the liquid storage tank, wherein a residual water content is 75 % or more as determined by a measuring method defined below with respect to liquid volumes in wells at four corners of the well plate mounted on the holder section, the measuring method comprising:

placing an SBS standard 96-well plate with all wells filled with 200 μL each of water in the main body, followed by leaving the well plate to stand with the liquid storage tank storing water, and with its lid being closed; and determining a change ratio of water content with respect to an initial value by formula (1) after leaving the well plate to stand for 20 days:

$$(\text{Residual water content}) \, (\%) = B/A \times 100 \qquad\qquad (1)$$

wherein:

A indicates an average value of initial values of volumes of water present in a total of four wells located at the four corners of the well plate, and
B indicates an average value of volumes of water remaining in the four wells located at the four corners of the well plate.

[20] A cell culture kit comprising: the drying suppression device of any one of [1] to [19]; a well plate that is housed in the drying suppression device and is provided with a plurality of wells; cells housed inside the wells; and a medium filled inside the wells.

Description of the Reference Numeral

1, IX, 2, 2A, 2B, 3A, 3B, 4, 5, 6 Drying suppression device

10, 30A, 30B, 40, 50, 60 Main body

10a, 30a, 40a, 50a, 60a Vapor port

11, 31, 31A, 31B, 41, 51, 61 Liquid storage tank

11a Opening

11x, 51x, 321x, 521x Bottom surface

12, 32, 32A, 32B, 42, 52, 62 Holder section

12a, 52a Region

13, 53 Support section

20, 20X, 92 Lid

20a Ventilation hole

22, 112, 122, 312, 312A, 312B, 322, 412, 422, 512, 522, 612, 922 Side wall

25, 26 Porous member

V, V1 Vapor

90 Well plate

100, 100X, 200A, 200B, 300A, 300B, 400, 500, 600 Cell culture kit

111, 121, 311, 321, 411, 421, 511, 521, 611 Bottom plate

315, 317 Light source

318, 325, 425 Light-shielding means

415 Light-collecting window

615 Heating unit

911 Well

Air Air

CM Culture medium

L Liquid

UV Ultraviolet rays

Citation List

Patent Literature

Patent Document 1 : Japanese Patent Application Unexamined Publication No. 2000-236869

**Claims**

1. A drying suppression device comprising:

   a main body housing a well plate; and
   an openable/closable lid provided on the main body,
   the main body comprising:

     a holder section that holds the well plate;
     a liquid storage tank that stores liquid; and
     a support section that supports the holder while keeping the holder section away from an inner bottom surface of the liquid storage tank,

   wherein, with the lid being closed, an inner space surrounded by the liquid storage tank and the lid communicate with an outside space.

2. The drying suppression device according to claim 1, wherein at least one of the lid and the liquid storage tank has a ventilation hole connecting the inner space and the outside space.

3. The drying suppression device according to claim 2, wherein:

   the liquid storage tank has an opening that opens above the holder;
   the lid covers the opening; and
   the ventilation hole is provided in the lid.

4. The drying suppression device according to claim 3, which has a vapor port provided around the holder section as viewed in plan, through which a vapor of the liquid stored in the liquid storage tank is allowed to flow, wherein:

   the lid overlaps with the vapor port as viewed in plan; and
   the ventilation hole overlaps with a region where the well plate is held by the holder section as viewed in plan, while not overlapping with the vapor port as viewed in plan.

5. The drying suppression device according to any one of claims 1 to 4, wherein the holder section comprises:

   a bottom plate on which the well plate is placed; and
   a side wall provided on the bottom plate so as to surround a region holding the well plate.

6. The drying suppression device according to any one of claims 1 to 5, which has a vapor port provided around the holder section as viewed in plan, through which a vapor of the liquid stored in the liquid storage tank is allowed to flow, wherein the vapor port is provided with a porous member.

7. The drying suppression device according to claim 6, wherein the porous member closes the vapor port.

8. The drying suppression device according to any one of claims 1 to 7, wherein the main body has:

a light source that emits ultraviolet rays toward the inside; and
a light-shielding means provided so as to face the light source.

9. The drying suppression device according to any one of claims 1 to 8, wherein the support section of the main body is separable from the liquid storage tank and the holder section.

10. The drying suppression device according to any one of claims 1 to 9, which has a vapor port provided around the holder section as viewed in plan, through which a vapor of the liquid stored in the liquid storage tank is allowed to flow, wherein:

the holder section is positioned away from the side wall of the liquid storage tank as viewed in plan; and
the vapor port is a gap between the holder section and the side wall.

11. The drying suppression device according to any one of claims 1 to 10, which has a vapor port provided around the holder section as viewed in plan, through which a vapor of the liquid stored in the liquid storage tank is allowed to flow, wherein:

the vapor port is a through hole that penetrates the holder section in a thickness direction thereof; and
the through hole is provided outside a region of the holder section where the well plate is held.

12. The drying suppression device according to any one of claims 1 to 11, wherein the liquid storage tank has a heating unit.

13. The drying suppression device according to any one of claims 1 to 12, which is configured to allow inflow of $CO_2$ into the inner space at an inflow rate of 20 ppm/second or higher from an ambient atmosphere having a $CO_2$ concentration of 5 %.

14. A drying suppression device comprising:

a main body housing a well plate; and
an openable/closable lid provided on the main body,
the main body comprising:

a holder section that holds the well plate;
a liquid storage tank that stores liquid; and
a support section that supports the holder while keeping the holder section away from an inner bottom surface of the liquid storage tank,
wherein a residual water content is 75 % or more as determined by a measuring method defined below with respect to liquid volumes in wells at four corners of the well plate placed on the holder section, the measuring method comprising:

placing an SBS standard 96-well plate with all wells filled with 200 $\mu$L each of water in the main body, followed by leaving the well plate to stand with the liquid storage tank storing water, and with its lid being closed; and
determining a change ratio of water content with respect to an initial value by formula (1) after leaving the well plate to stand for 20 days:

$$\text{(Residual water content) (\%) = B/A x 100} \qquad (1)$$

wherein:

A indicates an average value of initial values of volumes of water present in a total of four wells located at the four corners of the well plate; and
B indicates an average value of volumes of water remaining in the four wells located at the four corners of the well plate.

**15.** A cell culture kit comprising:

the drying suppression device of any one of claims 1 to 14;
a well plate that is housed in the drying suppression device and is provided with a plurality of wells;
cells housed inside the wells; and
a medium filled inside the wells.

# FIG. 1

1 (100)

FIG. 2

EP 3 878 937 A1

FIG. 3

EP 3 878 937 A1

FIG. 4

EP 3 878 937 A1

FIG. 5

FIG. 6

EP 3 878 937 A1

FIG. 7

# FIG. 8

EP 3 878 937 A1

FIG. 9

EP 3 878 937 A1

FIG. 10

EP 3 878 937 A1

FIG. 11

EP 3 878 937 A1

FIG. 12

FIG. 13

## FIG. 14

## FIG. 15

Europäisches Patentamt
European Patent Office
Office européen des brevets

## EUROPEAN SEARCH REPORT

Application Number

EP 21 15 9321

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 99/51347 A2 (PHARMACOPEIA INC [US]; BURBAUM JONATHAN [US]; SKWISH STEPHEN [US]) 14 October 1999 (1999-10-14) | 1,10, 13-15 | INV.<br>C12M1/32<br>C12M1/00 |
| A | * page 5, lines 12-32; figure 2 *<br>----- | 2-4 | C12M1/09<br>C12M1/12 |
| A | US 2016/003859 A1 (WENCZEL GYOERGY [AT] ET AL) 7 January 2016 (2016-01-07)<br>* paragraphs [0003], [0011], [0055], [0057], [0062], [0063]; figures 3,4,5A-5D *<br>----- | 1-15 | C12M1/34 |
| A | US 2003/235517 A1 (DEPPE HOLGAR [DE] ET AL) 25 December 2003 (2003-12-25)<br>* paragraphs [0031] - [0036]; figures 3-5 *<br>----- | 1-15 | |

TECHNICAL FIELDS
SEARCHED (IPC)

C12M
B01L

The present search report has been drawn up for all claims

| Place of search | Date of completion of the search | Examiner |
|---|---|---|
| Berlin | 31 July 2021 | Pantelidis, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 15 9321

This annex lists the patent family members relating to the patent documents cited in the above-mentioned European search report.
The members are as contained in the European Patent Office EDP file on
The European Patent Office is in no way liable for these particulars which are merely given for the purpose of information.

31-07-2021

| Patent document cited in search report | | Publication date | Patent family member(s) | | | Publication date |
|---|---|---|---|---|---|---|
| WO 9951347 | A2 | 14-10-1999 | AU | 3549399 | A | 25-10-1999 |
| | | | US | 5908776 | A | 01-06-1999 |
| | | | WO | 9951347 | A2 | 14-10-1999 |
| US 2016003859 | A1 | 07-01-2016 | CH | 708820 | A1 | 15-05-2015 |
| | | | CN | 104969075 | A | 07-10-2015 |
| | | | DE | 202014105173 | U1 | 10-02-2015 |
| | | | EP | 2943797 | A1 | 18-11-2015 |
| | | | JP | 6050546 | B2 | 21-12-2016 |
| | | | JP | 2016516206 | A | 02-06-2016 |
| | | | US | 2016003859 | A1 | 07-01-2016 |
| | | | WO | 2015067529 | A1 | 14-05-2015 |
| US 2003235517 | A1 | 25-12-2003 | AU | 8403101 | A | 02-04-2002 |
| | | | DE | 10046224 | A1 | 28-03-2002 |
| | | | EP | 1318869 | A1 | 18-06-2003 |
| | | | JP | 2004508842 | A | 25-03-2004 |
| | | | KR | 20030059144 | A | 07-07-2003 |
| | | | US | 2003235517 | A1 | 25-12-2003 |
| | | | WO | 0224336 | A1 | 28-03-2002 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000236869 A